# NEUE EUROPÄISCHE PATENTSCHRIFT

(11) **EP 0 412 366 B2**
(45) Veröffentlichungstag und Bekanntmachung des Hinweises auf die Entscheidung über den Einspruch: **31.10.2001**
(45) Hinweis auf die Patenterteilung: 28.09.1994
(21) Anmeldenummer: 90114340.4
(22) Anmeldetag: 26.07.1990
(51) Int. Cl.: C07D 307/28

(54) **Verfahren zur Herstellung von 2,5-Dihydrofuranen**
Process for the preparation of 2,5-dihydrofurans
Procédé pour la préparation de 2,5-dihydrofurannes

(30) Priorität: 08.08.1989 DE 3926147
(43) Veröffentlichungstag der Anmeldung: 13.02.1991
(73) Patentinhaber: BASF Aktiengesellschaft, 67063 Ludwigshafen (DE)
(72) Erfinder: Fischer, Martin, Dr., D-6700 Ludwigshafen (DE)

(56) Entgegenhaltungen:
- US-A- 3 812 158
- US-A- 3 932 468
- US-A- 3 996 248

## Beschreibung

Die vorliegende Erfindung betrifft ein Verfahren zur Herstellung von 2,5-Dihydrofuranen der allgemeinen Formel I 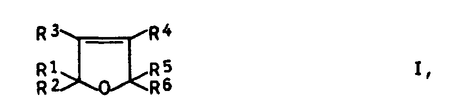 in der die Reste R¹, R², R³, R⁴, R⁵ und R⁶ gleich oder verschieden sind und für Wasserstoff oder C₁- bis C₄-Alkylgruppen stehen, durch die katalytische Umlagerung von 3,4-Epoxi-1-butenen der allgemeinen Formel II 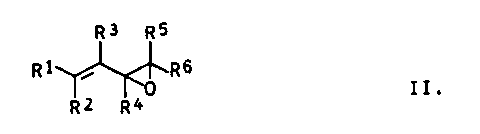 2,5-Dihydrofuran ist nach US-A 3 812 158 durch die Isomerisierung von Vinyloxiran unter der Einwirkung von Quecksilbersalzen erhältlich. Abgesehen davon, daß bei diesem Verfahren 2,5-Dihydrofuran nur in maximal 33 % Ausbeute gewonnen werden kann, steht der industriellen Verwertung dieses Verfahrens die Verwendung der toxischen Quecksilbersalzkatalysatoren entgegen.

US-A 3 932 468 beschreibt die durch übergangsmetallkomplexe und Brom- oder Jodwasserstoffgas katalysierte Umlagerung von Butadienmonoxid zu 2,5-Dihydrofuran. US-A 3 996 248 benötigt für diese Umlagerung ebenfalls Brom- oder Jodwasserstoff. Zur Verbesserung der Halogenwasserstoffkatalyse werden laut dieser Patentschrift homogen im Reaktionsmedium gelöste Lewis-Säuren, wie Zink-, Aluminium-, Bor-, Zinn- oder Magnesiumverbindungen, verwendet. weiterhin wird dem Reaktionssystem Kaliumjodid zugesetzt.

Nachteilig an diesen beiden Verfahren ist, daß infolge der Anwesenheit von Halogenwasserstoffen im Reaktionsmedium bei der destillativen Isolierung des 2,5-Dihydrofurans erhebliche Verluste auftreten und das erhaltene Dihydrofuran durch Halogenwasserstoff und/oder Jod verunreinigt ist. So fällt die Ausbeute an 2,5-Dihydrofuran nach den Angaben von US-A 3 932 468 von 78 % im Rohprodukt (bestimmt mittels Gaschromatographie) auf 58 % nach der Destillation des Produktes ab, wobei das Destillat nur einen Dihydrofurangehalt von 89 % hat. Dieses Verfahren ist daher unwirtschaftlich.

Weitere Gründe für die Unwirtschaftlichkeit dieser Verfahren sind die Korrosionsprobleme, welche durch die Verwendung von Halogenwasserstoffkatalysatoren verursacht werden, sowie der hohe Verbrauch an Lösungsmitteln und Katalysatoren, welche nicht zurückgeführt werden können.

Es bestand daher die Aufgabe ein Verfahren zu finden, das es erlaubt, 2,5-Dihydrofurane wirtschaftlich, in guten Ausbeuten und hoher Reinheit herzustellen, ohne mit den Nachteilen der geschilderten Verfahren behaftet zu sein.

Dementsprechend wurde ein Verfahren zur Herstellung von 2,5-Dihydrofuranen der allgemeinen Formel I 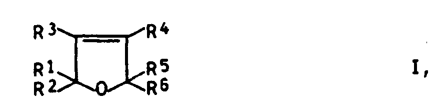 in der die Reste R¹, R², R³, R⁴, R⁵ und R⁶ gleich oder verschieden sind und für Wasserstoff oder C₁- bis C₄-Alkylgruppen stehen, durch die katalytische Umlagerung von 3,4-Epoxi-1-butenen der allgemeinen Formel II 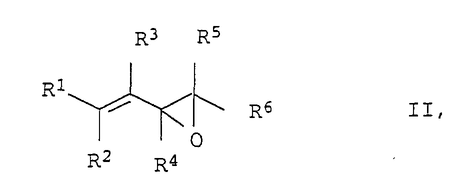 gefunden, das dadurch gekennzeichnet ist, daß man die Umlagerung ohne einen Halogenwasserstoffzusatz mittels eines Katalysatorsystems, welches die Komponenten A, B und C umfaßt, bei Temperaturen von 60 bis 200°C katalysiert, worin die Katalysatorkomponente
A ein Alkalimetall-, Erdalkalimetall- oder Oniumhalogenid ist,
B ein organisches Solubilisierungsmittel für die Komponente A ist, ausgenommen Kalium Jodid in Kombination mit N-Methylpyprrolidon und Zinn(IV)Chlorid.
C eine Lewis-Säure oder elementares Jod ist,
mit der Maßgabe, daß mindestens eine der Komponenten A oder C ein Jodid ist, ausgenommen Kalium Jodid in Kombination mit N-Methylpyrrolidon und Zinn(IV)Chlorid.

Das erfindungsgemäße Verfahren wird somit ohne einen Halogenwasserstoffzusatz ausgeführt.

Die nach dem erfindungsgemäßen Verfahren herstellbaren 2,5-Dihydrofurane können unsubstituiert sein oder 1 bis 6, vorzugsweise 1 bis 3 C₁- bis C₄-Alkylsubstituenten tragen.

Als Komponenten A des Katalysatorsystems können Alkalimetall-, Erdalkalimetall- oder Oniumhalogenide verwendet werden. Als Alkalimetall- oder Erdalkalimetallkomponenten kann jedes der Alkali- oder Erdalkalimetalle Anwendung finden, vorzugsweise werden im erfindungsgemäßen Verfahren jedoch Natrium- und Kaliumhalogenide benutzt.

Als Oniumkomponente eignen sich Ammonium-, Phosphonium- als auch Arsoniumionen, bevorzugt werden Ammoniumionen. Diese Oniumionen können 1 bis 4 organische Reste tragen, insbesondere C₁- bis C₂₀-Alkylgruppen und/oder Arylgruppen. Geeignete Oniumionen sind z.B.: Tetramethylammonium, Tetrabutylammonium, Octyltrimethylammonium, Benzyltrimethylammonium, Phenyltrimethylammonium, Tetramethylphosphonium, Tetrabutylphosphonium, Tetraphenylphosphonium.

Als Halogenkomponente der Katalysatorkomponente A können sämtliche Halogenide benutzt werden, vorausgesetzt die Katalysatorkomponente C enthält Jodid. Besonders bevorzugt ist als Halogenkomponente allerdings Jodid.

Es versteht sich von selbst, daß als Komponente A sowohl ein einzelnes der genannten Salze als auch Mischungen dieser Salze eingesetzt werden können. Bevorzugt werden aber die einzelnen Salze verwendet.

Die Komponente B des Katalysatorsystems ist, da sie als Solubilisierungsmittel für die Komponente A dient, der Komponente A so anzupassen, daß die jeweiligen Salze A im Reaktionsmedium, d.h. insbesondere im Alkenyloxiran II sowie in dessen Mischungen mit dem Dihydrofuran I, wie sie im Laufe der Umsetzung entstehen, gelöst werden. Da an die Komponente B, außer den Eigenschaften die Komponente A in Lösung zu bringen und sich ansonsten unter den Reaktionsbedingungen stabil und inert zu verhalten, im Prinzip keine weiteren Anforderungen bezüglich ihrer chemischen Eigenschaften gestellt werden müssen, können als Komponente B eine Vielzahl von Substanzen verwendet werden. Beispielsweise können als Solubilisierungsmittel B im erfindungsgemäßen Katalysatorsystem Kronenether, Kryptanden, Podanden, Polypodanden, Sphäranden oder Lariatether, welche die Kationen der Salze A mehr oder weniger spezifisch komplexieren und auf diese Weise im Reaktionsmedium löslich machen, angewandt werden. Solche Komplexbildner und ihre Eignung als solubilisierende Agentien für die Salze A werden beispielsweise beschrieben in Topics of Current Chemistry 101, 1-82, 147-200 (1981); Am. Chem. Soc. Symp. Ser. 326, 24 (1985); J. Am. Chem. Soc. 107, 3645 (1985) sowie in Kontakte (Merck) 1973 (3) 36; 1977 (1) 11; 1977 (2), 16 und 1983 (1), 38.

Als Solubilisierungsmittel für die Salze A können aber auch dipolar-aprotische Lösungsmittel verwendet werden.

Als Solubilisierungsagentien B bevorzugte Kronenether sind beispielsweise 12-Krone-4, 15-Krone-5, 18-Krone-6, Benzo-15-krone-5, Dibenzo-18-krone-6, Dibenzo-30-krone-10. Im Hinblick auf die in der oben angeführten Literatur beschriebenen kationenselektiven Komplexierungs- und somit auch Solubilisierungseigenschaften dieser Kronenether versteht es sich von selbst, daß sich beispielsweise 12-Krone-4 besonders durch Solubilisierung von Lithiumsalzen A und 15-Krone-5 bzw. 18-Krone-6 besonders zur Solubilisierung von Natrium- bzw. Kaliumsalzen A eignen, während sie zur Solubilisierung anderer Alkalimetallkationen nicht oder weniger gut geeignet sind. Bei der Anwendung von Kronenethern als Solubilisierungsmittel erweist es sich deshalb als vorteilhaft, die Art des verwendeten Kronenethers an das oder die in der Komponente A vorliegenden Kationen anzupassen, d.h. solche Kronenether als Komponente B auszuwählen, welche gegenüber den in der Komponente A vorhandenen Kationen die besten Komplexierungs- und folglich auch Solubilisierungseigenschaften haben.

Entsprechendes gilt im Hinblick auf die Anpassung der Art des verwendeten Kryptanden an das in der Komponente A vorliegende Kation, falls als Solubilisierungsmittel B die ebenfalls als kationenselektive Komplexierungs- und Solubilisierungsmittel wirkenden Kryptanden, beispielswseise [2.1.1]-Kryptand, [2.2. 1]-Kryptand oder [2.2.2]-Kryptand, verwendet werden.

Podanden, zu denen hier auch Polyethylenglykole, die aus mindestens 5 und bis zu 200 Oxiethylen-Einheiten aufgebaut sind oder solche Polyethylenglykole, welche durch Co- oder Blockpolymerisation von Ethylen- und Propylenoxid hergestellt worden sind, sowie deren Mono- und Diether mit C₁ - bis C₂₀-Alkoholen und Phenolen oder Alkylphenolen, gezählt werden, sind ebenfalls als Solubilisierungsmittel B geeignet. Zwar sind diese Polyglykole weniger selektiv und auch nicht so effektiv als Komplexbildner für die Kationen der Salze A wirksam wie die oben beschriebenen Kronenether und Kryptanden und müssen aus diesem Grunde in größeren Mengen pro Mol eingesetztem A zur Reaktionsmischung gegeben werden, dieser Nachteil wird aber durch die bessere Verfügbarkeit und den viel geringeren Preis dieser Podanden, verglichen mit den Kronenethern und Kryptanden, mehr als wettgemacht.

Als dipolar-aprotische Lösungsmittel, welche zur Solubilisierung der Katalysatorkomponente A im Reaktionsmedium geeignet sind, seien an dieser Stelle beispielhaft Tetramethylharnstoff, cyclische Harnstoffe wie N,N-Dimethylethylen- oder N,N-Dimethylpropylenharnstoff, Phosphorsäuretriamide wie Hexamethylphosphorsäuretriamid, N-Methylpyrrolidon, Dimethylformamid, Dimethylacetamid, Sulfolan oder Dimethylsulfoxid, Tetrahydrofuran, Dioxan und Dimethoxyethan genannt.

Selbstverständlich können auch mehrere der genannten Solubilisierungsmittel nacheinander oder in Mischungen zur Solublilisierung der Katalysatorkomponente A zum Reaktionsansatz gegeben werden. Des weiteren können auch noch andere unter den Reaktionsbedingungen inerte Lösungsmittel wie Diethylether, Ester wie Ethylacetat, Ketone wie Aceton oder aromatische Lösungsmittel wie Toluol oder Xylol zur Verdünnung des Reaktionsansatzes verwendet werden.

Als Komponente C des Katalysatorsystems dient eine Lewis-Säure oder elementares Jod. Vermutlich wirkt das Jod in diesem Falle ähnlich den Lewis-Säuren als Elektronenakzeptor, doch ist dies, da das Schicksal des Jodmoleküls im Zuge der Umsetzung nicht verfolgt wurde, eine bloße Vermutung und schließt eine andere Reaktionsweise des Jods nicht aus.

Als Lewis-Säuren können die üblicherweise in der präparativen organischen Chemie verwendeten Lewis-Säuren wie Zink-, Zirkonium-, Titan-, Nickel- oder Zinnhalogenide verwendet werden. Zwar werden im erfindungsgemäßen Verfahren die Lewis-Säuren vorzugsweise in Form ihrer Halogenide eingesetzt, doch ist auch die Verwendung von halogenfreien Lewis-Säuren, z.B. Dialkylzinnoxiden, möglich. Enthält die Katalysatorkomponente A kein Jodid, so sollte die eingesetzte Lewis-Säure C ein Jodid sein. Besonders bevorzugt werden im erfindungsgemäßen Verfahren die Lewis-Säuren Zinkchlorid, Zinkbromid und Zinkjodid benutzt.

Die Katalysatorkomponenten A, B und C werden üblicherweise im Reaktor vorgelegt und dazu das umzusetzende 3,4-Epoxi-1-buten (Vinyloxiran) gepumpt. Diese Zugabereihenfolge ist jedoch nicht kritisch und kann umgestellt werden.

Die Katalysatorkomponenten A, B und C werden im allgemeinen, bezogen auf eingesetztes Vinyloxiran, in den folgenden Mengen eingesetzt:

| | |
|---|---|
| Komponente A | 0,01 bis 5 Gew.%, vorzugsweise 0,5 bis 1 Gew.% |
| Komponente B | 0,05 bis 300 Gew.%, vorzugsweise 1 bis 100 Gew.% |
| Komponente C | 0,02 bis 10 Gew.%, vorzugsweise 0,5 bis 2 Gew.%. |

Bei kleineren Katalysatormengen nimmt die Reaktionsgeschwindigkeit ab; höhere Katalysatorzusätze als die hier aufgeführten sind nicht kritisch.

Die Komponente A wird bezüglich der Komponente B zweckmäßigerweise in einem Gewichtsverhältnis A:B von 1:1 bis 1:50, und bezüglich der Komponente C im allgemeinen in einem Gewichtsverhältnis A:C von 1:0,1 bis 1:10 verwendet. Die Komponente B wird zweckmäßigerweise mindestens in solchen Mengen zur Reaktionsmischung gegeben, daß der größte Teil der Komponente A von ihr bei der Reaktionstemperatur solubilisiert werden kann. Wie bereits erwähnt, müssen die Komponenten B, welche die Kationen der Salze A weniger gut solvatisieren, beispielsweise die Polyethylenglykolether, naturgemäß in größeren Mengen eingesetzt werden als z.B. die stark komplexierend wirkenden Kronenether. Da die zur Solubilisierung der Komponente A notwendige Menge des Solubilisierungsmittels B stark stoffabhängig ist, empfiehlt es sich, die optimale Menge an zuzusetzendem B in einem einfachen Vorversuch zu ermitteln.

Ähnliches gilt, wenn als Komponente A Oniumhalogenide verwendet werden, welche inhärent eine gewisse Löslichkeit im organischen Reaktionsmedium des Verfahrens haben. Insbesondere bei den vierfach alkyl- oder arylsubstituierten Oniumhalogeniden kann die Löslichkeit im Reaktionsmedium so groß sein, daß praktisch auf den Zusatz des Solubiliserungsmittels B verzichtet werden kann. Ein solches Vorgehen ist der beanspruchten Verfahrensweise äquivalent.

Das erfindungsgemäße Verfahren wird im allgemeinen bei Temperaturen von 60 bis 200°C, vorzugsweise bei 90 bis 180°C durchgeführt. Je nach Art und Menge des verwendeten Katalysatorsystems und in Abhängigkeit von der Reaktionstemperatur werden im allgemeinen Reaktionszeiten von wenigen Minuten bis zu 24 Stunden für eine vollständige Umsetzung benötigt.

Das erfindungsgemäße Verfahren wird im allgemeinen im Autoklaven unter dem Eigendruck des Reaktionssystems Vinyloxiran/Dihydrofuran und gegebenenfalls vorhandener Lösungsmittel durchgeführt. Der Druck im Reaktionsgefäß kann aber auch durch Aufpressen von Inertgasen, wie Stickstoff, über den Eigendruck des Reaktionssystems hinaus erhöht werden.

Die Umsetzung kann diskontinuierlich im Rührautoklaven oder kontinuierlich in Kaskaden-, Rohr- oder Schlaufenreaktoren erfolgen. Das gebildete 2,5-Dihydofuran kann aus dem Reaktionsaustrag destillativ isoliert werden. Das so erhaltene 2,5-Dihydrofuran enthält keine Verunreinigungen durch Jod oder Jodwasserstoff und kann ohne zusätzliche Reinigung weiterverarbeitet werden. Das als Katalysatorkomponente verwendete Jod bzw. die Jodide bleiben bei der Destillation als ionogenes Jod im schwerflüchtigen

Destillationsrückstand, der, da er sämtliche Katalysatorbestandteile enthält, gegebenenfalls nach Ergänzung der Komponenten A und C vorteilhaft als Katalysator wiederverwendet werden kann.

Die als Ausgangsmaterialien verwendeten Vinyloxirane II sind nach der Methode von Kadesch (J. Am. Chem. Soc. 68, 41 (1946)) erhältlich.

Die nach dem erfindungsgemäßen Verfahren erhältlichen 2,5-Dihydrofurane können nach üblichen Verfahren zu den entsprechenden Tetrahydrofuranen hydriert werden, welche als Lösungsmittel sowie als Monomere zur Herstellung von Polytetrahydrofuranen dienen.

### Beispiel 1

1,5 g Kaliumjodid, 2,9 g Zinkjodid und 2,4 g 18-Krone-6 wurden in einem 300 ml Rührautoklaven unter Stickstoff auf 150°C erhitzt. Zu dieser Mischung wurden innerhalb von 4 Stunden 100 g Vinyloxiran unter Rühren zudosiert. Nach Ende der Vinyloxiranzugabe wurde die Temperatur noch 1 Stunde auf 150°C gehalten. Nach dem Abkühlen wurde der Autoklaveninhalt in eine Destillationsapparatur umgefüllt und destilliert. Bei der Destillation wurden 90 g 2,5-Dihydrofuran mit einem Reinheitsgrad von 98 % erhalten (Ausbeute: 90 %). Im Sumpf der Destillationsapparatur verblieben 16,8 g schwerflüchtiger Destillationsrückstand, der einen Gehalt von 15 Gew.% an ionogenem Jod hatte.

### Beispiel 2

Der Destillationsrückstand aus Beispiel 1 wird zusammen mit 0,3 g Kaliumjodid und 0,6 g Zinkjodid in einem 300 ml Rührautoklaven unter Stickstoff auf 150°C erhitzt. Die Umsetzung mit 100 g Vinyloxiran gemäß Beispiel 1 und analoge Aufarbeitung ergaben 91 g 2,5-Dihydrofuran (Ausbeute 91 %) und 26,7 g schwerflüchtigen Destillationsrückstand, welcher erneut als Katalysator verwendbar ist.

### Beispiel 3

1,0 g Kaliumjodid, 2,0 g Zinkjodid und 20 g Polyethylenglykoldimethylether mit einem mittleren Molekulargewicht von 2000 wurden mit 100 g Vinyloxiran in einem 300 ml Rührautoklaven 20 Stunden auf 110°C erhitzt, wobei 78 % des eingesetzten Epoxids umgesetzt wurden. Bei der Destillation des Reaktionsgemisches wurden 86,7 g eines Gemisches aus 25 Gew.% Vinyloxiran und 75 Gew.% 2,5-Dihydrofuran erhalten (Umsatz: 78 %, Selektivität: 83 %).

### Beispiel 4

1,0 g Kaliumjodid, 1,9 g Zinkjodid, 19 g Polyethylenglykoldimethylether eines mittleren Molekulargewichts von 2000 und 50 ml Tetrahydrofuran wurden in einem 300 ml Rührautoklaven auf 110°C erhitzt. Innerhalb von 12 Stunden wurden 50 g Vinyloxiran zudosiert und anschließend nochmals 2 Stunden bei 110°C gerührt. Die Destillation des Reaktionsaustrages ergab 46,5 g 2,5-Dihydrofuran (Ausbeute: 93 %) im Gemisch mit 44,4 g Tetrahydrofuran.

### Beispiel 5

50 g Vinyloxiran, 2,7 g 18-Krone-6, 0,5 g Kaliumjodid und 0,7 g Zinkbromid wurden 8 Stunden unter Eigendruck bei 120°C im Rührautoklaven gerührt. Die gaschromatographische Analyse des Reaktionsaustrages mit Chlorbenzol als internem Standard ergab einen Gehalt von 70,5 % Vinyloxiran und 15 % 2,5-Dihydrofuran, was einer Selektivität von 75 % bei 22 % Umsatz entspricht.

### Beispiel 6

1,3 g Kaliumjodid, 2,1 g 18-Krone-6, 2,0 g Jod und 50 ml Tetrahydrofuran wurden in einem 300 ml Autoklaven auf 150°C erhitzt. Innerhalb von 3 Stunden wurden 87 g Vinyloxiran zudosiert und anschließend die Reaktionstemperatur noch 1 Stunde auf 150°C gehalten. Die gaschromatographische Analyse des Reaktionsaustrages ergab einen Gehalt von 20 % Vinyloxiran und 23,3 % 2,5-Dihydrofuran, was einer Selektivität von 56 % bei 68 % Umsatz entspricht.

### Beispiel 7

2,6 g Kaliumjodid, 6 g Zinkjodid und 45 g N-Methylpyrrolidon wurden in einem 300 ml Rührautoklaven auf 110°C erhitzt. Innerhalb von 5 Stunden wurden 100 g Vinyloxiran zudosiert und anschließend die Reaktionstemperatur noch 1 Stunde auf 110°C gehalten. Bei der anschließenden Destillation wurde das 2,5-Dihydrofuran in einer Ausbeute von 88 % erhalten.

### Beispiel 8

Der Versuch von Beispiel 1 wurde wiederholt mit dem einzigen Unterschied, daß das Kaliumjodid gegen 1,4 g Natriumjodid ausgetauscht wurde. Nach der gaschromatographischen Analyse waren im Reaktionsaustrag 91 % 2,5-Dihydrofuran entstanden.

### Beispiel 9

Der Versuch von Beispiel 1 wurde wiederholt, wobei anstelle von Vinyloxiran 1-Methyl-2-vinyloxiran verwendet wurde. 2-Methyl-2,5-dihydrofuran bildete sich nach dem Ergebnis der gaschromatographischen Analyse in einer Ausbeute von 75 %.

### Beispiel 10

Ein Gemisch aus 5 g Vinyloxiran, 50 mg Kaliumjodid, 250 mg 18-Krone-6 und 100 mg Zirkoniumtetrachlorid wurde in einem Glasautoklaven 8 Stunden auf 110°C erhitzt. Der Reaktionsaustrag enthielt laut quantitativer Analyse des NMR-Spektrums 48 Gew.% Vinyloxiran und 26 Gew.% 2,5-Dihydrofuran.

### Beispiel 11

52 g Vinyloxiran, 0,6 g Kaliumjodid, 1,0 g 18-Krone-6 und 2 g Dibutylzinnoxid wurden in einem Rührautoklaven 8 Stunden auf 150°C erhitzt. Die gaschromatographische Analyse des Reaktionsaustrages ergab einen Gehalt von 23,8 Gew.% Vinyloxiran und 35,1 Gew.% 2,5-Dihydrofuran.

### Beispiel 12

5 g Vinyloxiran, 250 mg 18-Krone-6, 50 mg Kaliumjodid und 200 mg Zinntetrajodid wurden in einem Glasautoklaven 16 Stunden auf 120°C erhitzt. Dabei entstanden laut gaschromatographischer Analyse 34 Gew.% 2,5-Dihydrofuran.

## Patentansprüche

1. Verfahren zur Herstellung von 2,5-Dihydrofuranen der allgemeinen Formel I 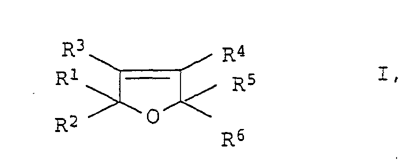 in der die Reste R¹, R², R³, R⁴, R⁵ und R⁶ gleich oder verschieden sind und für Wasserstoff oder C₁- bis C₄-Alkylgruppen stehen, durch die katalytische Umlagerung von 3,4-Epoxi-1-butenen der allgemeinen Formel II 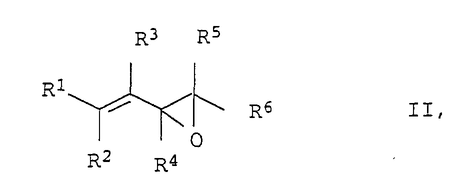 **dadurch gekennzeichnet, daß** man die Umlagerung ohne einen Halogenwasserstoffzusätz mittels eines Katalysatorsystems, welches die Komponenten A, B und C umfaßt, bei Temperaturen von 60 bis 200°C katalysiert, worin die Katalysatorkomponente
A ein Alkalimetall-, Erdalkalimetall- oder Oniumhalogenid ist,
B ein organisches Solubilisierungsmittel für die Komponente A ist,
C eine Lewis-Säure oder elementares Jod ist,
mit der Maßgabe, daß mindestens eine der Komponenten A oder C ein Jodid ist, ausgenommen Kalium Jodid in Kombination mit N-Methylpyrrolidon und Zinn(IV)Chlorid.

2. Verfahren nach Anspruch 1, **dadurch gekennzeichnet, daß** man die Katalysatorkomponente A in Mengen von 0,01 bis 5 Gew.-%, die Katalysatorkomponente B in Mengen von 0,05 bis 300 Gew.-% und die Katalysatorkomponente C in Mengen von 0,02 bis 10 Gew.-%, jeweils bezogen auf das Gewicht des eingesetzten 3,4-Epoxi-1-butens II, verwendet.

3. Verfahren nach Anspruch 1, **dadurch gekennzeichnet, daß** man die Katalysatorkomponente A bezüglich der Katalysatorkomponennte B in einem Gewichtsverhältnis A:B von 1:1 bis 1:50 und bezüglich der Katalysatorkomponente C in einem Gewichtsverhältnis A:C von 1:1 bis 1:10 einsetzt.

4. Verfahren nach Anspruch 1, **dadurch gekennzeichnet, daß** man als Katalysatorkomponente B Kronenether, Kryptanden, Podanden, Polyethylenglykole und/oder dipolar-aprotische Lösungsmittel verwendet.

5. Verfahren nach Anspruch 1, **dadurch gekennzeichnet, daß** man als Katalysatorkomponente C ein Zinkhalogenid verwendet.

6. Verfahren zur Herstellung von 2,5-Dihydrofuranen der allgemeinen Formel 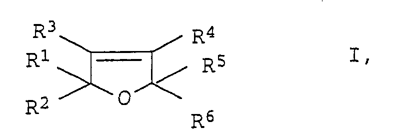 in der die Reste R¹, R², R³, R⁴' R⁵ und R⁶ gleich oder verschieden sind und für Wasserstoff oder C₁- bis C₄-Alkylgruppen stehen, durch die katalytische Umlagerung von 3,4-Epoxi-1-butenen der allgemeinen Formel II 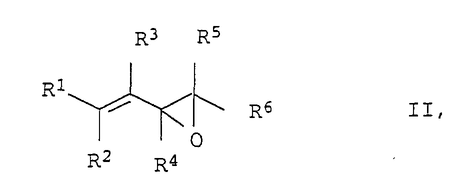 **dadurch gekennzeichnet, daß** man die Umlagerung mittels eines Katalysatorsystems, welches aus den Komponenten A und C besteht, bei Temperaturen von 60 bis 200°C katalysiert, wobei A ein Oniumhalogenid ist, das im Reaktionsmedium löslich ist, C eine Lewis-Säure oder elementares Jod ist, mit der Maßgabe, daß mindestens eine der Komponenten A oder C ein Jodid ist.

## Claims

1. A process for the preparation of 2,5-dihydrofurans of the general formula I 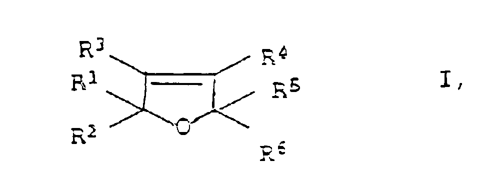 where R¹, R², R³, R⁴, R⁵ and R⁶ are identical or different and are hydrogen or C₁-C₄-alkyl, by the catalytic rearrangement of 3,4-epoxy-1-butenes of the general formula II 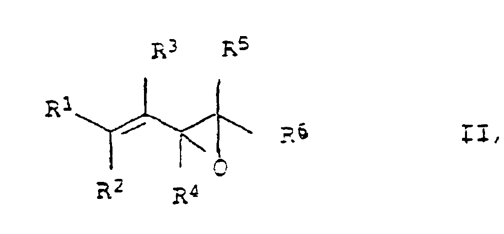 which comprises the rearrangement being catalyzed without the addition of halogen halide by a system which contains components A, B and C, at from 60 to 200°C, where
A is a halide of an alkali metal or alkaline earth metal or an onium halide,
B is an organic solubilizer for component A, and
C is a Lewis acid or elemental iodine,
with the proviso that at least one of components A or C is an iodide, with the exception of potassium iodide in combination with N-methylpyrrolidone and tin(IV) chloride.

2. A process as claimed in claim 1, wherein catalyst component A is used in amounts of from 0.01 to 5% by weight, catalyst component B is used in amounts of from 0.05 to 300% by weight, and catalyst component C is used in amounts of from 0.02 to 10% by weight, in each case based on the weight of 3,4-epoxy-1-butene II.

3. A process as claimed in claim 1, wherein the ratio by weight of catalyst component A to catalyst component B is from 1:1 to 1:50 and of catalyst component A to catalyst component C is from 1:1 to 1:10.

4. A process as claimed in claim 1, wherein crown ethers, cryptands, podands, polyethylene glycols and/or dipolar aprotic solvents are used as catalyst component B.

5. A process as claimed in claim 1, wherein a zinc halide is used as catalyst component C.

6. A process for the preparation of 2,5-dihydrofurans of the general formula 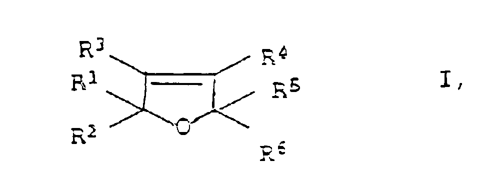 where R¹, R², R³, R⁴, R⁵ and R⁶ are identical or different and are hydrogen or C₁-C₄-alkyl, by the catalytic rearrangement of 3,4-epoxy-1-butenes of the general formula II 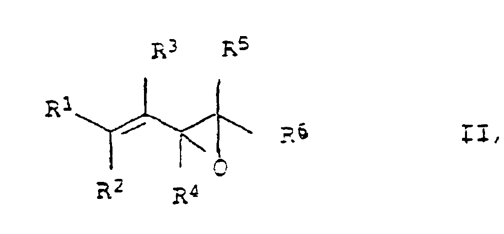 which comprises the rearrangement being catalyzed by a system comprising components A and C, at from 60 to 200°C, where A is an onium halide which is soluble in the reaction medium and C is a Lewis acid or elemental iodine, with the proviso that at least one of components A or C is an iodide.

## Revendications

1. Procédé de préparation de 2,5-dihydrofurannes de formule générale I 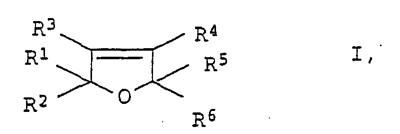 dans laquelle les radicaux R¹, R², R³, R⁴, R⁵ et R⁶ sont identiques ou différents et représentent chacun un atome d'hydrogène ou un groupe alkyle en C₁ à C₄, par transposition catalytique de 3,4-époxy-1-butènes de formule générale II 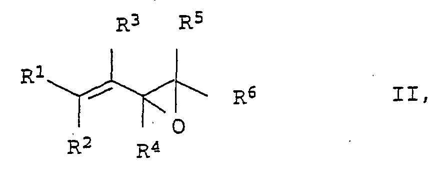 **caractérisé en ce qu'**on catalyse la transposition, sans addition d'un halogénure d'hydrogène, à l'aide d'un système catalyseur comportant les composants A, B et C, à des températures de 60 à 200°C, où le composant catalyseur est
A un halogénure d'un métal alcalin, d'un métal alcalino-terreux ou d'onium,
B un agent solubilisateur organique du composant A,
C un acide de Lewis ou de l'iode élémentaire,
à la condition qu'au moins l'un des composants A ou C soit un iodure, à l'exception de l'iodure de potassium en combinaison avec la N-méthylpyrrolidone et le chlorure d'étain(IV).

2. Procédé selon la revendication 1, **caractérisé en ce qu'**on utilise le composant catalyseur A en des quantités de 0,01 à 5 % en poids, le composant catalyseur B en des quantités de 0,05 à 300 % en poids et le composant catalyseur C en des quantités de 0,02 à 10 % en poids, toujours par rapport au poids du 3,4-époxy-1-butène II utilisé.

3. Procédé selon la revendication 1, **caractérisé en ce qu'**on utilise le composant catalyseur A en une quantité par rapport au composant catalyseur B correspondant à un rapport pondéral A:B de 1:1 à 1:50 et par rapport au composant catalyseur C correspondant à un rapport pondéral A:C de 1:1 à 1:10.

4. Procédé selon la revendication 1, **caractérisé en ce qu'**on utilise en tant que composant catalyseur B des éthers-couronnes, des kryptands, des podands, des polyéthylèneglycols et/ou des solvants aprotiques dipolaires.

5. Procédé selon la revendication 1, **caractérisé en ce qu'**on utilise en tant que composant catalyseur C un halogénure de zinc.

6. Procédé de préparation de 2,6-dihydrofurannes de formule générale 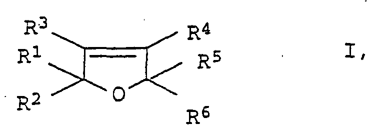 dans laquelle les radicaux R¹, R², R³, R⁴, R⁵ et R⁶ sont identiques ou différents et représentent chacun un atome d'hydrogène ou un groupe alkyle en C₁ à C₄, par transposition catalytique de 3,4-époxy-1-butènes de formule générale II 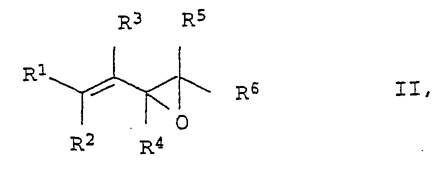 **caractérisé en ce qu'**on catalyse la transposition à l'aide d'un système catalyseur constitué des composants A et C à des températures de 60 à 200°C, où A est un halogénure d'onium qui est soluble dans le milieu réactionnel, C est un acide de Lewis ou de l'iode élémentaire, à la condition qu'au moins l'un des composants A ou C soit un iodure.
